# EUROPEAN PATENT SPECIFICATION

(11) **EP 2 679 257 B1**
(45) Date of publication and mention of the grant of the patent: **17.10.2018**
(21) Application number: 12750266.4
(22) Date of filing: 23.02.2012
(51) Int. Cl.: A61M 1/14, A61M 1/34

(54) **BLOOD PURIFICATION DEVICE**
BLUTREINIGUNGSVORRICHTUNG
DISPOSITIF DE PURIFICATION DU SANG

(30) Priority: 23.02.2011 JP 2011037336
(43) Date of publication of application: 01.01.2014
(73) Proprietor: Asahi Kasei Medical Co., Ltd., Chiyoda-ku Tokyo 101-8101 (JP)
(72) Inventor: OKAZAKI, Soichiro, Tokyo 101-8101 (JP)
(74) Representative: dompatent von Kreisler Selting Werner - Partnerschaft von Patent- und Rechtsanwälten mbB
(86) International application number: PCT/JP2012/054417
(87) International publication number: WO 2012/115189

(56) References cited:
- EP-A2- 0 263 384
- EP-A2- 0 820 776
- WO-A1-2008/152809
- JP-A- S 645 563
- JP-A- 3 261 480
- JP-A- S5 911 865
- US-A- 4 637 880

## Description

### BACKGROUND

The present invention relates to a blood purification device . Also disclosed is a cleaning method for the blood purification device.

Blood purification therapies are therapeutic methods in which the blood of a patient is taken out from the body and returned to the body after being purified by blood purifiers, and typical examples of the blood purification therapies include a dialysis therapy (Patent Document 1). In the dialysis therapy, the blood of a patient is come in contact with dialysis fluid via a semi-permeable membrane, and undesired substances are discharged from the blood of the patient to the side of the dialysis fluid using a diffusion phenomenon caused by a difference in concentration between substances. Large-molecular-weight undesired substances, which cannot be removed only by the diffusion phenomenon, are removed by filtration. Besides the dialysis therapy, various blood purification therapies for intractable autoimmune disease are covered by insurance. For example, the cases of colitis ulcerosa, Crohn's disease, chronic rheumatism, etc., can be dramatically improved by the removal of white blood cells from the blood of a patient (Patent Document 2). Some of various blood purifiers used in these blood purification therapies separate substances using their adsorption performance besides substance separation performance such as the diffusion and the filtration described above.

Since the blood purification therapies adopt a therapeutic mode in which the blood of a patient is taken out from the body and returned to the body after being purified by blood purifiers, they require at least one or more hours, normally several hours, or several tens of hours depending on the circumstances. Particularly, examples of the blood purification therapies requiring a long therapeutic time include continuous slow type blood purification therapies (Patent Document 3). As the continuous slow type blood purification therapies, various therapeutic modes such as a continuous hemofiltration therapy, a continuous hemodialysis therapy, and a continuous hemodiafiltration therapy (continuous renal replacement therapy (CRRT)) are available and applied to therapies for renal disease, failure of multiple organs, etc., accompanied by serious cardiovascular complications in the field of emergent and intensive therapies. As referred to as the "continuous slow type," any of the therapies require several days or one or more weeks in the long term for their single therapy to gradually perform blood purification.

Patent Document 1: Patent Publication JP-A-2007-268257
Patent Document 2: Patent Publication JP-A-2010-63761
Patent Document 3: Patent Publication JP-A-2006-025813
Patent Document 4: Patent Publication JP-B-63-28626
Patent Document 5: Patent Publication JP-B-5-80909

US 4,637,880 and EP 0820776 each disclose: (A) a plurality of blood purifiers parallelly connected to the blood circuit; (B) switch means capable of selectively supplying the blood to the plurality of blood purifiers; and (C) cleaning fluid supply means capable of selectively supplying cleaning fluid to the plurality of blood purifiers.

In the blood purification therapies requiring a long therapeutic time, it is important to maintain the removing performance of blood purifiers over the entire therapeutic time. For example, in order to sufficiently demonstrate the substance separation performance of filtration, it is desired that filtration membranes loaded in blood purifiers be maintained at their initial use state at all times. However, from the viewpoint of the principle of the blood purification therapies in which the blood of a patient is taken out from the body and returned to the body again after being purified by the blood purifiers, the blood purification therapies require at least one or more hours, normally several hours, or several tens of hours depending on the circumstances, which makes it difficult to maintain the filtration membranes at their initial use state. As a result, there may be a problem in which the performance of the blood purifiers is gradually degraded in the middle of the therapies.

Particularly, since the continuous slow type blood purification therapies described above require several days or one or more weeks in the long term, they cause not only the gradual degradation of the blood purifiers in the middle of the therapies but also the clogging of the filtration membranes, etc., of the blood purifiers due to blood coagulation. Therefore, it is required to interrupt the therapies during the therapeutic times and replace the blood purifiers. The average service lives of the blood purifiers are about 24 hours in a normal case, but the blood purifiers are used up for about four hours in a short case, which requires the replacement of the blood purifiers several or more times during their single therapy. Therefore, heavy burdens are placed on doctors and nurses, and particularly the replacement at night increases the burdens. In addition, since the nurses are required to stay at all times so as to respond to the replacement at night, labor costs are increased, which results in the high-costs of the blood purification therapies in hospital management. Moreover, the larger the frequency of replacing the blood purifiers, the longer the time required for the replacement becomes and the larger the burdens on patients become.

### SUMMARY

The present invention has been made in view of the above points and has an object of maintaining the removing performance of blood purifiers over the entire therapeutic time and preventing the gradual degradation of the blood purifiers in the middle of a therapy. The present invention has another object of reducing burdens on doctors and nurses in a long blood purification therapy such as the interruption of the therapy due to the clogging of blood purifiers and the replacement operation of the blood purifiers and of reducing burdens on patients while attaining the cost reduction of the blood purification therapy.

In order to attain the above objects, the present invention provides:
A blood purification device having a blood circuit that is configured to supply blood taken out from a body to blood purifiers and to return the blood purified by the blood purifiers to the body, comprising:
a plurality of blood purifiers parallelly connected to the blood circuit; switch means configured to selectively supply the blood to the plurality of blood purifiers; and
cleaning fluid supply means configured to selectively supply cleaning fluid to the plurality of blood purifiers,
characterized in that
   each blood purifier comprises a filtration membrane A with a primary side and a secondary side and wherein the blood purifiers are connected to the blood circuit such that the blood is supplied to the primary sides of filtration membranes (A) of the blood purifiers, and
   the cleaning fluid supply means is configured to supply the cleaning fluid to the secondary sides of the filtration membranes (A) of the blood purifiers, wherein the cleaning fluid is the supplemental fluid to be supplied to the blood during blood purification.

According to the present invention, it is possible to supply, while supplying the blood to one of the blood purifiers of the blood circuit to perform blood purification, the cleaning fluid to the other of the blood purifiers to clean the same. By the switch of the blood purifier to which the blood is to be supplied and the blood purifier to which the cleaning fluid is to be supplied one from the other, it is possible to successively clean the blood purifiers while continuing the blood purification to prevent the coagulation of the blood in the blood purifiers. Thus, it is possible to maintain the performance of the blood purifiers in the middle of a therapy, eliminate the need to frequently replace the blood purifiers, reduce burdens on doctors and nurses in the blood purification therapy, and reduce burdens on patients while attaining the cost reduction of the blood purification therapy.

Also disclosed is a cleaning method for a blood purification device having a plurality of blood purifiers, an upstream circuit for supplying blood collected from a living body to a branch part, a downstream circuit for returning the blood at a merge part to the living body, a plurality of branch circuits branching off from a branch part of the upstream circuit toward the respective blood purifiers and merging with each other at a merge part of the downstream circuit via the respective blood purifiers, and cleaning fluid supply means capable of selectively supplying cleaning fluid to the blood purifiers of the plurality of branch circuits, the cleaning method including a step of operating, while the blood is supplied from the upstream circuit to the blood purifier of one of the branch circuits and the blood having passed through the blood purifier is caused to flow into the downstream circuit, the cleaning fluid supply means to supply the cleaning fluid to the blood purifier of the other one of the branch circuits to be fed to the branch part and the merge part via either of the branch circuits.

In the cleaning method for the blood purification device, the cleaning fluid may be supplied to secondary sides of purification membranes of the blood purifiers, caused to flow out to primary sides via the purification membranes, and fed to the branch part and the merge part.

In addition, the blood may be repeatedly supplied to the plurality of blood purifiers in a predetermined order, and the cleaning fluid may be supplied to the blood purifier to which the blood has been supplied in a previous order.

The cleaning method for the blood purification device may include a step of temporarily increasing a supply flow rate of the cleaning fluid in the step of supplying the cleaning fluid to the blood purifier of the other one of the branch circuits.

In the cleaning method, the blood purification device may be a continuous slow type blood purification device.

According to the present invention, it is possible to maintain the removing performance of blood purifiers over the entire therapeutic time and prevent the gradual degradation of the performance of the blood purifiers in the middle of a therapy. Moreover, it is possible to reduce the clogging of the mechanism of a circuit and remarkably improve the service life of the entire system. As a result, the number of interruption times is dramatically decreased in a long time therapy such as a continuous slow type blood purification therapy, whereby burdens on nurses and patients are remarkably reduced and costs for the therapy can be sufficiently reduced.

Another effect of the present invention is that there is no need to worry about clogging due to coagulation since a plurality of blood purifiers is successively cleaned in the present invention. Accordingly, there is a possibility that a therapy can be continued without the introduction of an anticoagulant. In this case, since not only the mechanism of, for example, a syringe for applying the anticoagulant but also the replacement operation of the anticoagulant in the middle of the therapy are eliminated, it is possible to provide a simple and easy-to-operate blood purification device. Moreover, since there is no need to worry about clogging, a continuous hemofiltration (CHF) therapy, which produces a great effect but may suffer from clogging in a conventional method, can be positively implemented. Since it is expected that the CHF therapy produces a great effect with a less fluid rate, it is possible to attain both cost reduction due to a reduction in supplemental fluid rate and an improvement in therapeutic effect.

### DESCRIPTION OF DRAWINGS

FIG. 1 is an explanatory diagram showing the outline of the configuration of a blood purification device;
FIG. 2 is an explanatory diagram showing the state of the blood purification device in a case in which blood is supplied to a second blood purifier;
FIG. 3 is an explanatory diagram showing the configuration of the blood purification device in a case in which cleaning fluid supply means and drainage means are integrated;
FIG. 4 is an explanatory diagram showing the state of the blood purification device in a case in which the blood is supplied to the second purifier;
FIG. 5 is an explanatory diagram of the blood purification device showing other configuration examples of the cleaning fluid supply means and the drainage means;
FIG. 6 is an explanatory diagram showing the state of the blood purification device in a case in which the blood is supplied to the second blood purifier;
FIG. 7 is an explanatory diagram of the blood purification device in a case in which opening/closing valves are used as switch means; and
FIG. 8 is an explanatory diagram showing the state of the blood purification device in a case in which the blood is supplied to the second blood purifier.

### DETAILED DESCRIPTION

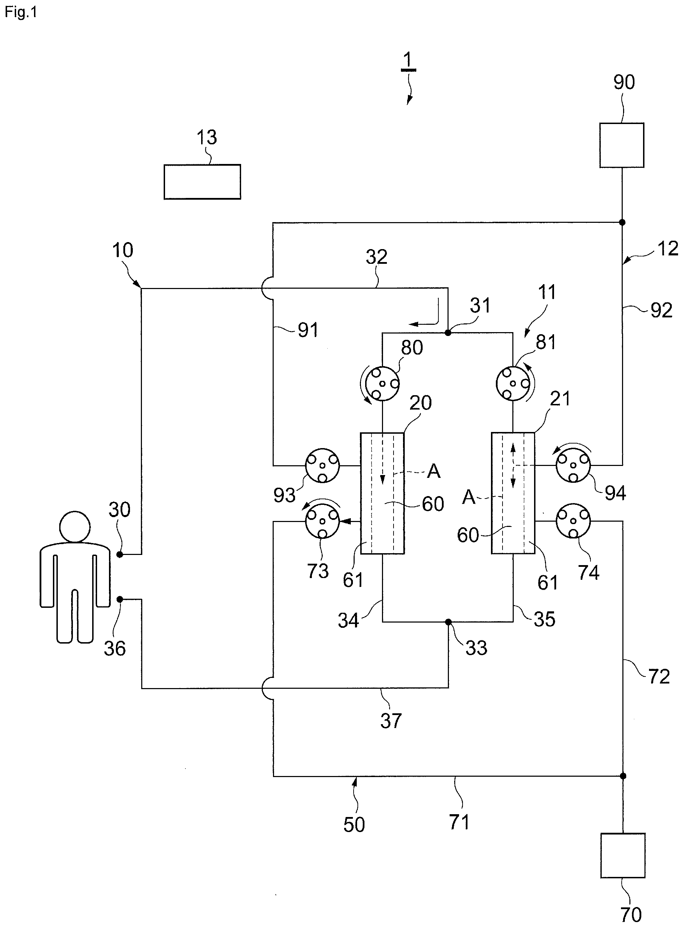

Hereinafter, a description will be given, with reference to the drawings, of preferred embodiments of the present invention. FIG. 1 is an explanatory diagram showing the outline of the configuration of a blood purification device (continuous slow type blood purification device) 1 according to this embodiment.

The blood purification device 1 has, for example, a blood circuit 10, switch means 11, cleaning fluid supply means 12, and a control unit 13.

The blood circuit 10 is, for example, a tube-like flow channel that supplies blood taken out from the body to a blood purifier and returns the blood purified by the blood purifier to the body. The blood circuit 10 has, for example, two blood purifiers 20 and 21 parallelly connected thereto.

More specifically, the blood circuit 10 has, for example, an upstream circuit 32 ranging from a blood collection puncture part 30 to a branch part 31, two branch circuits 34 and 35 ranging from the branch part 31 to a merge part 33, and a downstream circuit 37 ranging from the merge part 33 to an autotransfusion part 36.

The first and second branch circuits 34 and 35 are connected in parallel. The first branch circuit 34 has the first blood purifier 20, and the second branch circuit 35 has the second blood purifier 21.

The first and second blood purifiers 20 and 21 have, for example, a filtration membrane A serving as a hollow fiber purification membrane, and each of the branch circuits 34 and 35 is communicated with a primary side 60 of the filtration membrane A. The "primary side" represents the side of the filtration membrane into which blood is caused to flow prior to its purification. If the filtration membrane is, for example, a hollow fiber membrane, the primary side represents either the inside or outside of the hollow fiber.

The first and second blood purifiers 20 and 21 have drainage means 50. The drainage means 50 has a drainage bag 70, a discharge flow channel 71 ranging from a secondary side 61 of the filtration membrane A of the first blood purifier 20 to the drainage bag 70, and a discharge flow channel 72 ranging from a secondary side 61 of the filtration membrane A of the second blood purifier 21 to the drainage bag 70. The "secondary side" represents the side opposite to the primary side of the filtration membrane. If the filtration membrane is, for example, a hollow fiber membrane, the secondary side represents the outside of the hollow fiber provided that the primary side represents, for example, the inside of the hollow fiber while the primary side represents the inside of the hollow fiber provided that the secondary side represents, for example, the outside of the hollow fiber.

The discharge flow channels 71 and 72 have discharge pumps 73 and 74, respectively. When negative pressure is formed in the secondary sides 61 of the respective blood purifiers 20 and 21 by the discharge pumps 73 and 74, undesired substances inside the blood of the primary sides 60 are caused to pass through the filtration membranes A, whereby the blood is purified. The undesired substances flowing into the secondary sides 61 after having passed through the filtration membranes A are discharged into the drainage bag 70 via the discharge flow channels 71 and 72.

The blood of a patient delivered from the blood collection puncture part 30 into the blood circuit 10 is supplied under pressure to the sides of the blood purifiers 20 and 21 via the upstream circuit 32. The pressure supply of the blood of the patient to the blood purifiers 20 and 21 is controlled by, for example, blood pumps. The pressure supply of the blood of the patient is implemented by the control of the difference between a first pump 80 provided on the upstream side of the blood purifier 20 of the first branch circuit 34 and a second pump 81 provided on the upstream side of the second blood purifier 21 of the second branch circuit 35. More specifically, the driving flow rate of the first pump 80 is controlled to be, for example, at 80 ml/min for positive rotation and that of the second pump 81 is controlled to be, for example, at 20 ml/min for negative rotation, whereby a normal flow force (flow directed to the sides of the blood purifiers) of 60 ml/min as the difference between them is produced in the upstream circuit 32. Note that the pressure supply of the blood to the sides of the blood purifiers 20 and 21 may also be implemented in such a manner that a blood pump (not shown) is separately provided on the upstream circuit 32 and the tube of the upstream circuit 32 is squeezed by the blood pump.

The switch means 11 allows the blood to be selectively supplied to either the first blood purifier 20 or the second blood purifier 21 in, for example, the blood circuit 10 and has, for example, the first and second pumps 80 and 81 in this embodiment. The first pump 80 is provided on, for example, the upstream side of the first blood purifier 20 of the first branch circuit 34, and the second pump 81 is provided on, for example, the upstream side of the second blood purifier 21 of the second branch circuit 35. The first and second pumps 80 and 81 are tube pumps capable of rotating positively and negatively and allow fluid to be fed to the side of the merge part 33 in the forward direction and the side of the branch part 31 in the backward direction. The forward rotation of the first pump 80 allows the blood to be supplied to the side of the first blood purifier 20 of the first branch circuit 34, and the forward rotation of the second pump 81 allows the blood to be supplied to the side of the second blood purifier 21 of the second branch circuit 35.

The cleaning fluid supply means 12 has, for example, a supplemental fluid bag 90 in which supplemental fluid serving as cleaning fluid is stored, supplemental fluid flow channels 91 and 92 connected from the supplemental fluid bag 90 to the secondary sides 61 of the respective blood purifiers 20 and 21, and supplemental fluid pumps 93 and 94 provided on the respective supplemental fluid flow channels 91 and 92. The supplemental fluid is fluid used to replenish the blood with water inside the blood corresponding to an amount reduced with the removal of the undesired substances by the blood purifiers. By the operation of the supplemental fluid pumps 93 and 94, the supplemental fluid in the fluid bag 90 is allowed to be supplied to the secondary sides 61 of the blood purifiers 20 and 21 and then supplied from the secondary sides 61 to the primary sides 60 via the filtration membranes A. In addition, by the operation of the first pump 80, the supplemental fluid of the primary side 60 of the first blood purifier 20 is allowed to flow into either the upstream or downstream of the second branch circuit 35 or to flow into both of them via the first branch circuit 34. By the operation of the second pump 81, the supplemental fluid of the primary side 60 of the second blood purifier 21 is allowed to flow into either the upstream or downstream of the first branch circuit 34 or to flow into both of them via the second branch circuit 35.

The control unit 13 is allowed to control the operations of the discharge pumps 73 and 74, the first and second pumps 80 and 81, the supplemental fluid pumps 93 and 94, etc., to perform the blood purification process and the cleaning process of the blood purification device 1. For example, the control unit 13 is allowed to control the supply of the blood and that of the supplemental fluid such that the supplemental fluid is supplied to the second blood purifier 21 while the blood is supplied to the first blood purifier 20 and the supplemental fluid is supplied to the first blood purifier 20 while the blood is supplied to the second blood purifier 21. Note that the control unit 13 is composed of, for example, a micro computer having a CPU and a memory and allowed to perform the blood process and the cleaning process of the blood purification device 1 by the execution of a program recorded on the memory.

Next, a description will be given of an example of the operations of the blood purification device 1 configured as described above. In a continuous slow type blood purification process, the first and second pumps 80 and 81, the discharge pump 73, and the supplemental fluid pump 94 are operated as shown in FIG. 1. The first pump 80 is operated in the forward direction and the second pump 81 is operated in the backward direction such that the flow rate of the first pump 80 becomes greater than that of the second pump 81. Thus, the blood of the patient is supplied from the blood collection puncture part 30 to the first blood purifier 20 of the first branch circuit 34 via the upstream circuit 32. In the first blood purifier 20, undesired substances inside the blood are removed. The undesired substances are discharged by the discharge pump 73 into the drainage bag 70 via the discharge flow channel 71. The blood purified after having passed through the first blood purifier 20 is returned from the autotransfusion part 36 to the patient via the downstream circuit 37.

In addition, by the operation of the second pump 81 in the backward direction and the operation of the supplemental fluid pump 94 simultaneously with the supply of the blood to the first blood purifier 20, the supplemental fluid of the supplemental fluid bag 90 is supplied to the secondary side 61 of the second blood purifier 21. The supplemental fluid is caused to flow from the secondary side 61 into the primary side 60, fed to, for example, the branch part 31 and the merge part 33 via the second branch circuit 35, and delivered from both of the branch part 31 and the merge part 33 into either the upstream or downstream blood of the first branch circuit 34. By the supply of the supplemental fluid, the inside of the second blood purifier 21 is cleaned and water inside the blood corresponding to an amount reduced with the removal of the undesired substances by the first blood purifier 20 is replenished.

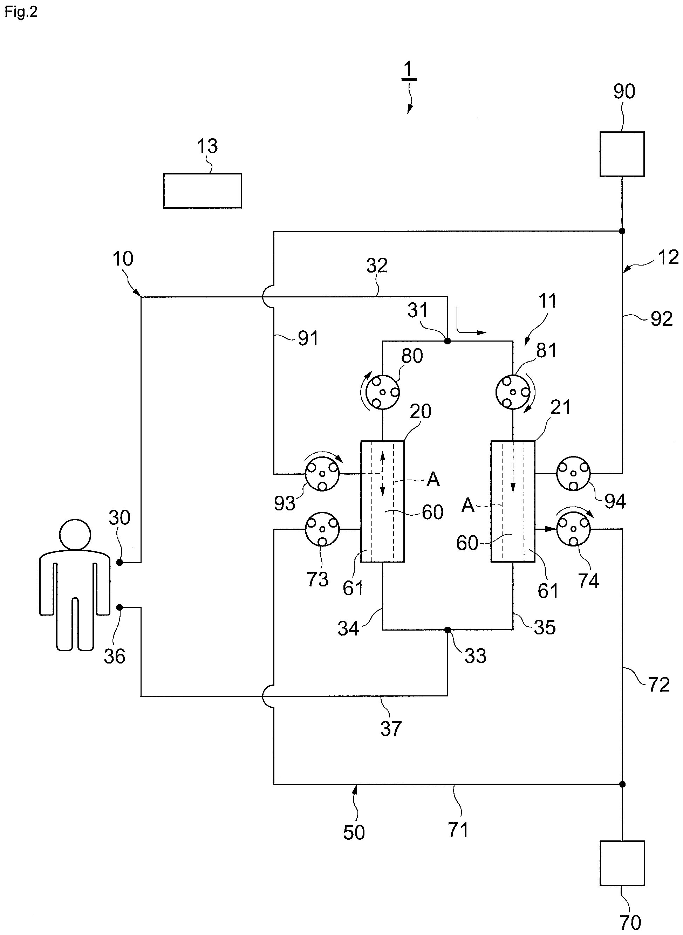

After lapse of a predetermined time, as shown in FIG. 2, the operations of the discharge pump 73 and the supplemental fluid pump 94 are stopped, the discharge pump 74 and the supplemental fluid pump 93 are operated, and the first and second pumps 80 and 81 are operated in the backward and forward directions, respectively. Thus, the blood of the patient is fed from the blood collection puncture part 30 to the second blood purifier 21 of the second branch circuit 35 via the upstream circuit 32, and undesired substances inside the blood are removed. The undesired substances are discharged into the drainage bag 70 via the discharge flow channel 72. The blood purified after having passed through the second blood purifier 21 is returned from the autotransfusion part 36 to the patient via the downstream circuit 37.

In addition, by the operation of the first pump 80 in the backward direction and the operation of the supplemental fluid pump 93 simultaneously with the supply of the blood to the second blood purifier 21, the supplemental fluid of the supplemental fluid bag 90 is supplied to the secondary side 61 of the first blood purifier 20. The supplemental fluid is caused to flow from the secondary side 61 into the primary side 60, fed to the branch part 31 and the merge part 33 via the first branch circuit 34, and flow from both of the branch part 31 and the merge part 33 into either the upstream or downstream blood of the second branch circuit 35. By the supply of the supplemental fluid, the first blood purifier 20 is cleaned and water inside the blood corresponding to an amount reduced with the removal of the undesired substances by the second blood purifier 21 is replenished.

The supply of the blood and that of the supplemental fluid to the first and second blood purifiers 20 and 21 as described above are alternately switched every, for example, 2 to 30 minutes, and the blood purification process is finished after lapse of a predetermined time.

According to this embodiment, it is possible to supply, while supplying the blood to one of the blood purifiers of the blood circuit 10 to perform blood purification, the supplemental fluid serving as the cleaning fluid to the other of the blood purifiers to clean the same. Further, by the switch of the blood purifier to which the blood is to be supplied and the blood purifier to which the cleaning fluid is to be supplied one from the other, it is possible to successively clean the blood purifiers 20 and 21 while performing the blood purification to prevent the coagulation of the blood in the blood purifiers 20 and 21. Thus, it is possible to eliminate the need to frequently replace the blood purifiers 20 and 21, reduce burdens on doctors and nurses in a blood purification therapy, and reduce burdens on patients while attaining the cost reduction of the blood purification therapy. In addition, by the repetitive cleaning of the blood purifiers 20 and 21 in a predetermined order, it is possible to significantly prolong the service lives of the respective blood purifiers 20 and 21.

The first and second blood purifiers 20 and 21 are connected to the blood circuit 10 such that the blood is supplied to the primary sides 60 of the filtration membranes A, and the cleaning fluid supply means 12 is capable of supplying the cleaning fluid to the secondary sides 61 of the blood purifiers 20 and 21. Thus, since the cleaning fluid is allowed to flow from the secondary sides 61 into the primary sides 60 in the direction opposite to the direction at filtration, it is possible to effectively remove extraneous matter from the filtration membranes A to efficiently clean the filtration membranes A. In addition, since the cleaning fluid flowing out to the primary sides 60 is fed to the branch part 31 and the merge part 33 via the corresponding branch circuits to be supplied into the blood, it is also possible to clean the upstream and downstream sides of the blood purifiers on the branch circuit with the cleaning fluid.

In addition, since the cleaning fluid is the supplemental fluid to be supplied to the blood at the blood purification, it is possible to clean the blood purifiers 20 and 21 while feeding the supplemental fluid to the blood to be subjected to the purification process. In this case, since there is no need to separately provide a cleaning fluid supply circuit besides the supplemental fluid circuit, the simplification of the configuration of the device and the cost reduction are attained. Specific examples of the fluid used as the cleaning fluid include, for example, Sublood (trademark) blood filtration replenishment fluid (Fuso Pharmaceutical Industries, Ltd.).

The blood circuit 10 has the first and second branch circuits 34 and 35, and the switch means 11 has the first and second pumps 80 and 81 capable of feeding fluid in the forward and backward directions in the respective branch circuits 34 and 35. Thus, it is possible to suitably switch the supply of the blood to the first and second branch circuits 34 and 35 by the first and second pumps 80 and 81.

Since the control unit 13 controls the supply of the blood and that of the cleaning fluid such that the cleaning fluid is supplied to the second blood purifier 21 while the blood is supplied to the first blood purifier 20 and the cleaning fluid is supplied to the first blood purifier 20 while the blood is supplied to the second blood purifier 21, it is possible to suitably switch the supply of the cleaning fluid and that of the blood.

Since the blood purification device 1 performs the continuous slow type blood purification process that comparatively requires a time, there is a significant advantage in eliminating the replacement of the blood purifiers or reducing the frequency of replacing the blood purifiers.

Although the supply flow rate of the cleaning fluid to the blood purifiers is constant in the above embodiment, it may be temporarily increased in the process of supplying the cleaning fluid. In this case, for example, the cleaning fluid supply means 12 is allowed to change the supply flow rate of the cleaning fluid to the blood purifiers 20 and 21 by the supplemental fluid pumps 93 and 94. For example, in a state in which the blood flows into the first blood purifier 20 and the cleaning fluid is supplied to the second blood purifier 21, the control unit 13 controls the supplemental fluid pump 94 of the cleaning fluid supply means 12 to temporarily increase the supply flow rate of the cleaning fluid to the second blood purifier 21. For example, during the supply of the cleaning fluid to the second blood purifier 21 for, for example, 2 to 30 minutes as a whole, the flow rate is normally at 10 to 20 ml/min. However, for 1 to 30 seconds for example, the flow rate is increased to 50 to 200 ml/min, which is five to ten times as large as the normal flow rate. In this manner, the cleaning performance of the cleaning fluid is enhanced, and the second blood purifier 21 is more properly cleaned. Note conversely that in a state in which the blood flows into the second blood purifier 21 and the cleaning fluid is supplied to the first blood purifier 20, the supply flow rate of the cleaning fluid to the first blood purifier 20 is temporarily increased.

According to this example, it is possible to enhance the cleaning performance with respect to the blood purifiers 20 and 21 and more effectively clean the blood purifiers 20 and 21 with the temporary increase in the supply flow rate of the cleaning fluid. In addition, since the supply flow rate of the cleaning fluid is temporarily increased, it is possible to limit a cost increase due to the increase in the supply rate of the cleaning fluid. Moreover, even if the cleaning fluid is supplied into the blood as the supplemental fluid, it is possible to continue a proper therapy without losing body fluid balance.

Note that in this example, the temporal increase in the supply flow rate of the cleaning fluid may be made a plurality of times at the supply of the cleaning fluid without being limited to once. In addition, the timing may be arbitrarily selected.

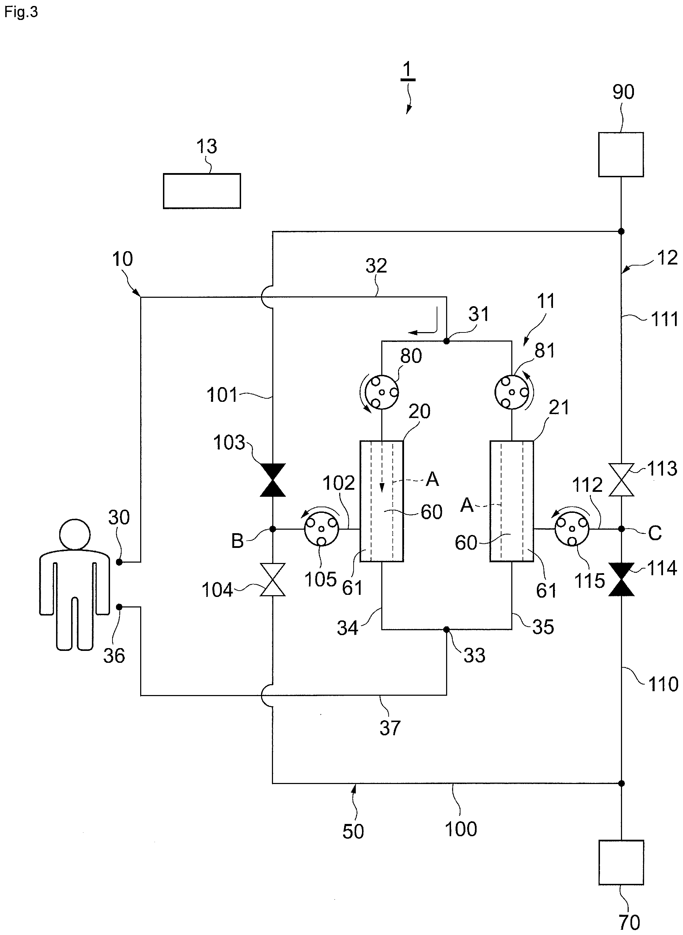

Although the cleaning fluid supply means 12 and the drainage means 50 are separate in the above embodiment, they may be integrated as shown in FIG. 3. Specifically, a discharge flow channel 100 connected to the drainage bag 70 and a supplemental fluid flow channel 101 connected to the supplemental fluid bag 90 are connected to each other, and a common flow channel 102 is connected between the connected part B and the secondary side 61 of the first blood purifier 20. The supplemental fluid flow channel 101 has an opening/closing valve 103, and the discharge flow channel 100 has an opening/closing valve 104. In addition, the common flow channel 102 has a pump 105.

Similarly, a discharge flow channel 110 connected to the drainage bag 70 and a supplemental fluid flow channel 111 connected to the supplemental fluid bag 90 are connected to each other, and a common flow channel 112 is connected between the connected part C and the secondary side 61 of the second blood purifier 21. The supplemental fluid flow channel 111 has an opening/closing valve 113, and the discharge flow channel 110 has an opening/closing valve 114. In addition, the common flow channel 112 has a pump 115.

Further, for example, when the blood is supplied to the first blood purifier 20, the opening/closing valve 103 is closed, the opening/closing valve 104 is opened, and the pump 105 is operated in the backward direction (in the direction of the drainage bag 70) as shown in FIG. 3. Thus, undesired substances inside the blood flowing into the secondary side 61 of the first blood purifier 20 are discharged into the drainage bag 70 via the common flow channel 102 and the discharge flow channel 100. While, on the side of the second blood purifier 21, the opening/closing valve 113 is opened, the opening/closing valve 114 is closed, and the pump 115 is operated in the forward direction (to the side of the second blood purifier 21). Thus, the supplemental fluid of the supplemental fluid bag 90 is supplied to the secondary side 61 of the second blood purifier 21 via the supplemental fluid flow channel 111 and the common flow channel 112, whereby the second blood purifier 21 is cleaned.

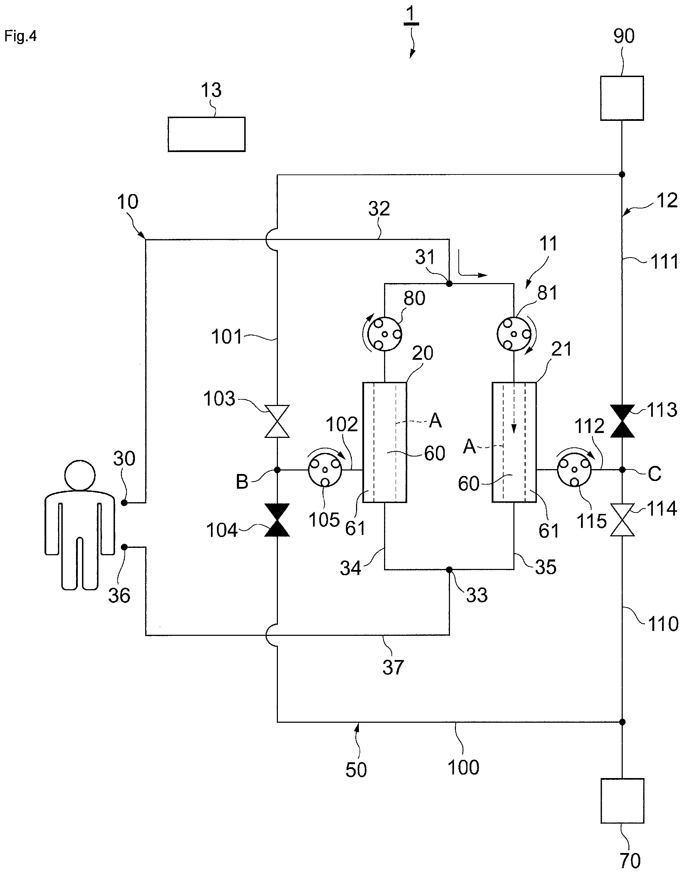

In addition, when the blood is supplied to the second blood purifier 21, the opening/closing valve 113 is closed, the opening/closing valve 114 is opened, and the pump 115 is operated in the backward direction (in the direction of the drainage bag 70) as shown in FIG. 4. Thus, undesired substances inside the blood flowing into the secondary side 61 of the second blood purifier 21 are discharged into the drainage bag 70 via the common flow channel 112 and the discharge flow channel 110. While, on the side of the first blood purifier 20, the opening/closing valve 103 is opened, the opening/closing valve 104 is closed, and the pump 105 is operated in the forward direction (to the side of the first blood purifier 20). Thus, the supplemental fluid of the supplemental fluid bag 90 is supplied to the secondary side 61 of the first blood purifier 20 via the supplemental fluid flow channel 101 and the common flow channel 102, whereby the first blood purifier 20 is cleaned. According to the above example, it is possible to simplify the circuit configurations of the cleaning fluid supply means 12 and the drainage means 50.

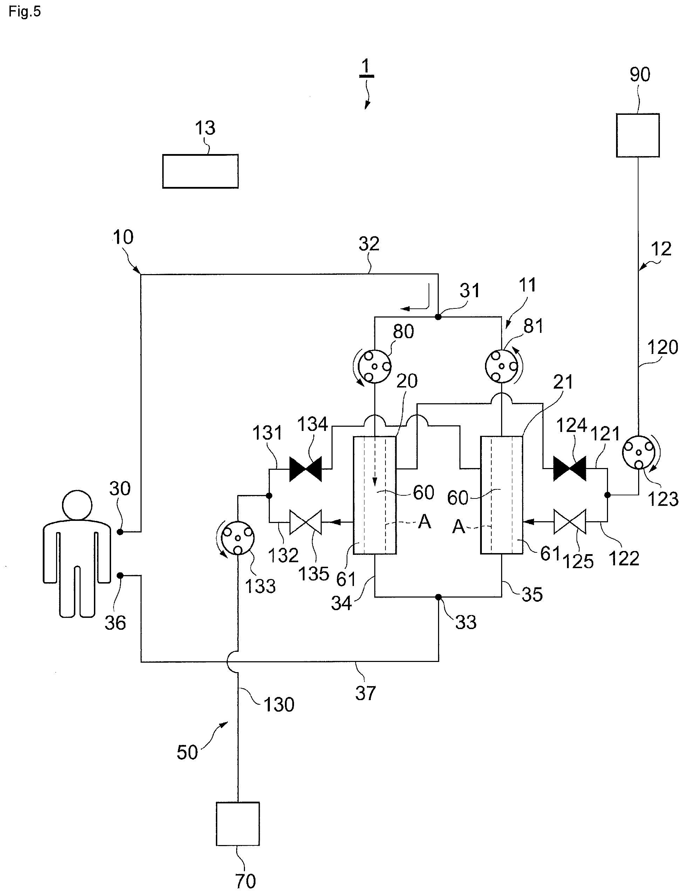

Moreover, as shown in FIG. 5, the cleaning fluid supply means 12 may have a common flow channel 120 connected to the supplemental fluid bag 90, and the common flow channel 120 may branch off into two pieces such that the two branch flow channels 121 and 122 are connected to the first and second blood purifiers 20 and 21, respectively. In this case, the common flow channel 120 has a supplemental fluid pump 123. The branch flow channels 121 and 122 have opening/closing valves 124 and 125, respectively. In addition, the drainage means 50 may have a common flow channel 130 connected to the drainage bag 70, and the common flow channel 130 may branch off into two pieces such that the two branch flow channels 131 and 132 are connected to the first and second blood purifiers 20 and 21, respectively. In this case, the common flow channel 130 has a pump 133. The branch flow channels 131 and 132 have opening/closing valves 134 and 135, respectively.

Further, for example, when the blood is supplied to the first blood purifier 20, the opening/closing valve 135 is opened, the opening/closing valve 134 is closed, and the pump 133 is operated as shown in FIG. 5. Thus, undesired substances inside the blood flowing into the secondary side 61 of the first blood purifier 20 are discharged into the drainage bag 70 via the branch flow channel 132 and the common flow channel 130. While, on the side of the second blood purifier 21, the opening/closing valve 125 is opened, the opening/closing valve 124 is closed, and the pump 123 is operated. Thus, the supplemental fluid of the supplemental fluid bag 90 is supplied to the secondary side 61 of the second blood purifier 21 via the common flow channel 120 and the branch flow channel 122, whereby the second blood purifier 21 is cleaned.

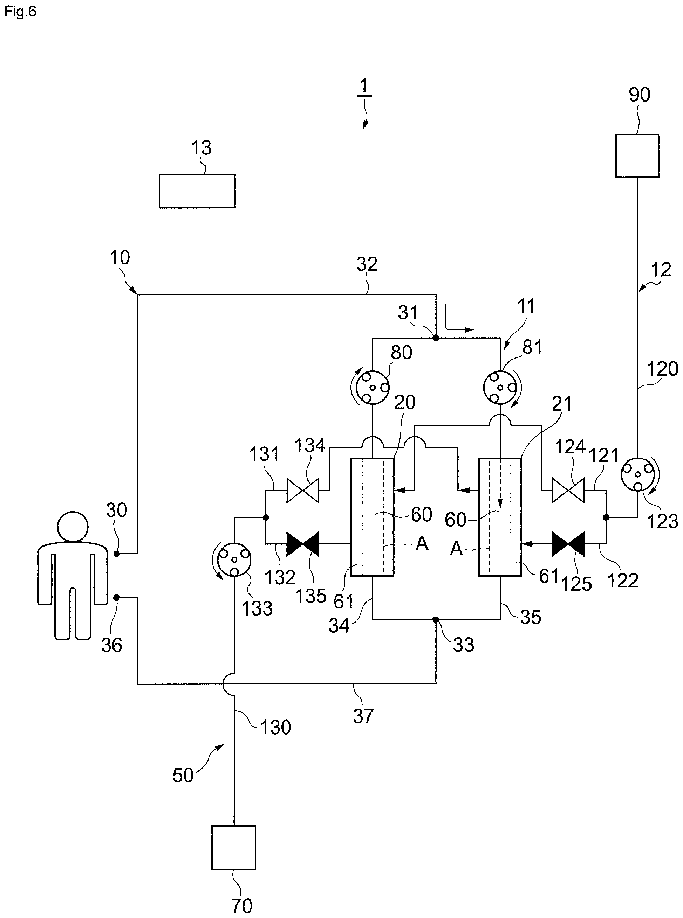

In addition, when the blood is supplied to the second blood purifier 21, the opening/closing valve 135 is closed, the opening/closing valve 134 is opened, and the pump 133 is operated as shown in FIG. 6. Thus, undesired substances inside the blood flowing into the secondary side 61 of the second blood purifier 21 are discharged into the drainage bag 70 via the branch flow channel 131 and the common flow channel 130. While, on the side of the first blood purifier 20, the opening/closing valve 124 is opened, the opening/closing valve 125 is closed, and the pump 123 is operated. Thus, the supplemental fluid of the supplemental fluid bag 90 is supplied to the secondary side 61 of the first blood purifier 20 via the common flow channel 120 and the branch flow channel 121, whereby the first blood purifier 20 is cleaned. According to the above example, it is possible to further simplify the circuit configurations of the cleaning fluid supply means 12 and the drainage means 50.

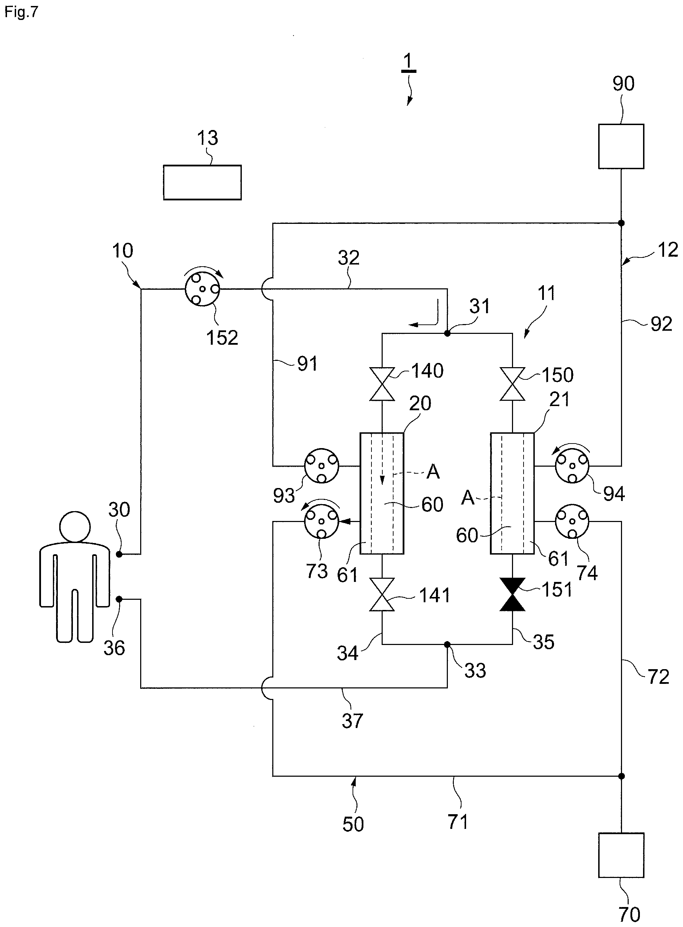

In the above embodiments, the switch means 11 has the first and second pumps 80 and 81. However, as shown in FIG. 7, the switch means 11 may have opening/closing valves 140 and 141, respectively, provided on the upstream and downstream sides of the first blood purifier 20 of the first branch circuit 34, opening/closing valves 150 and 151, respectively, provided on the upstream and downstream sides of the second blood purifier 21 of the second branch circuit 35, and a blood pump 152 provided on the upstream circuit 32.

In this case, for example, when the blood is supplied to the first blood purifier 20, the opening/closing valves 140 and 141 are opened, and either the opening/closing valve 150 or the opening/closing valve 151 is closed (the opening/closing valve 151 is closed in Fig. 7). Thus, on the side of the first blood purifier 20, the blood is caused to flow into the primary side 60 by the blood pump 152, whereby the blood purification process is performed. On the side of the second blood purifier 21, the supplemental fluid is supplied to the secondary side 61 by the supplemental fluid pump 94. The supplemental fluid flows from the primary side 60 of the second blood purifier 21 to the side of the opened opening/closing valve 150, whereby the second blood purifier 21 is cleaned.

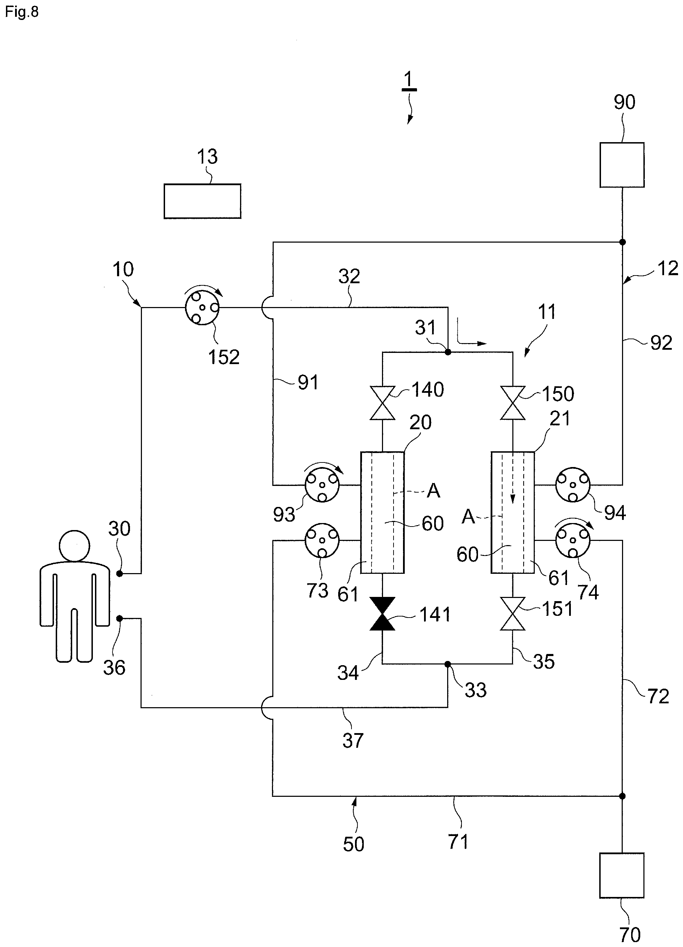

In addition, when the supply of the blood is switched to supply the blood to the second blood purifier 21, the opening/closing valves 150 and 151 are opened and either the opening/closing valve 140 or the opening/closing valve 141 is closed (the opening/closing valve 141 is closed in FIG. 8) as shown in FIG. 8. Thus, on the side of the second blood purifier 21, the blood flows into the primary side 60 by the blood pump 152, whereby the blood purification process is performed. On the side of the first blood purifier 20, the supplemental fluid is supplied to the secondary side 61 by the supplemental fluid pump 93. The supplemental fluid flows from the primary side 60 of the first blood purifier 20 to the side of the opened opening/closing valve 140, whereby the first blood purifier 20 is cleaned.

The preferred embodiments of the present invention are described above with reference to the accompanying drawings.

For example, even if the circuit configurations of the blood circuit 10, the cleaning fluid supply means 12, and the drainage means 50 in the above embodiments are replaced by other circuit configurations, the present invention is applicable. Also, the configuration of the switch means 11 may be different. The above embodiments describe the example of the case in which the two blood purifiers are provided. However, the number of blood purifiers may be arbitrarily selected provided that a plurality of blood purifiers is provided. If three or more blood purifiers are provided, the blood process and the cleaning may be performed on the blood purifiers in, for example, a predetermined order. After the round of the blood process and the cleaning on the blood purifiers, the blood process and the cleaning may be returned to the first blood purifier again to be performed in the predetermined order. In addition, the above embodiments describe the blood purification device 1 that performs a continuous hemofiltration therapy, but the present invention is applicable to any devices that perform various continuous slow type blood purification therapies such as a continuous hemodialysis therapy and a continuous hemodiafiltration therapy. Note that in performing continuous dialysis, it may be possible to use, for example, the supplemental fluid as dialysis fluid, supply the supplemental fluid to the secondary side of the blood purifier into which the blood is caused to flow, discharge the supplemental fluid via the discharge flow channels 71 and 72 to dialyze the blood. In addition, it may be possible to apply the present invention to any blood purification processes other than the continuous slow type blood purification therapy.

- 1: blood purification device
- 10: blood circuit
- 11: switch means
- 12: cleaning fluid supply means
- 13: control unit
- 20: first blood purifier
- 21: second blood purifier
- 30: blood collection puncture part
- 31: branch part
- 32: upstream circuit
- 33: merge part
- 34: first branch circuit
- 35: second branch circuit
- 36: autotransfusion part
- 37: downstream circuit
- 50: drainage means
- 60: primary side
- 61: secondary side
- 70: drainage bag
- 71, 72: discharge flow channel
- 73, 74: discharge pump
- 80: first pump
- 81: second pump
- 90: supplemental fluid bag
- 91, 92: supplemental fluid flow channel
- 93, 94: supplemental fluid pump
- 100, 110: discharge flow channel
- 101, 111: supplemental fluid flow channel
- 102, 112: common flow channel
- 103, 104, 113, 114: opening/closing valve
- 105, 115: pump
- 120, 130: common flow channel
- 121, 122, 131, 132: branch flow channel
- 124, 125, 134, 135: opening/closing valve
- 123: supplemental fluid pump
- 133: pump
- 140, 141, 150, 151: opening/closing valve
- 152: blood pump
- A: filtration membrane
- B, C: connected part

## Claims

1. A blood purification device (1) having a blood circuit (10) that is configured to supply blood taken out from a body to blood purifiers (20, 21) and to return the blood purified by the blood purifiers (20, 21) to the body, comprising:
a plurality of blood purifiers (20, 21) parallelly connected to the blood circuit (10);
switch means (11) configured to selectively supply the blood to the plurality of blood purifiers (20, 21); and
cleaning fluid supply means (12) configured to selectively supply cleaning fluid to the plurality of blood purifiers (20, 21),
**characterized in that**
each blood purifier (20, 21) comprises a filtration membrane A with a primary side (60) and a secondary side (61) and wherein the blood purifiers (20, 21) are connected to the blood circuit (10) such that the blood is supplied to the primary sides (60) of filtration membranes (A) of the blood purifiers (20, 21), and
the cleaning fluid supply means (12) is configured to supply the cleaning fluid to the secondary sides (61) of the filtration membranes (A) of the blood purifiers (20, 21), wherein the cleaning fluid is a supplemental fluid to be supplied to the blood during blood purification.

2. The blood purification device (1) according to claim 1, wherein
the blood circuit (10) comprises a plurality of branch circuits (34, 35) branching off on an upstream side of the blood circuit (10) to be connected to the respective blood purifiers (20, 21) and merging with each other on a downstream side of the blood circuit (10) via the respective blood purifiers (20, 21), and
the switch means (11) comprises a pump (80, 81) configured to feed fluid in forward and backward directions in the respective branch circuits (34, 35).

3. The blood purification device (1) according to claim 1 or 2, wherein the plurality of blood purifiers (20, 21) is a first and a second blood purifiers (20, 21) and wherein the blood circuit (10) has the first and the second blood purifiers (20, 21) parallelly connected thereto,
the blood purification device (1) further comprising a control unit (13) configured to control the supply of the blood and the supply of the cleaning fluid such that the cleaning fluid is supplied to the second blood purifier (21) while the blood is supplied to the first blood purifier (20) and the cleaning fluid is supplied to the first blood purifier (20) while the blood is supplied to the second blood purifier (21).

4. The blood purification device (1) according to any of claims 1 to 3, wherein the cleaning fluid supply means (12) is configured to change the supply flow rate of the cleaning fluid to the blood purifiers (20, 21).

5. The blood purification device (1) according to any of claims 1 to 4, wherein the plurality of blood purifiers (20, 21) is a first and a second blood purifiers (20, 21) and wherein
the blood circuit (10) has the first and the second blood purifier (20, 21) parallelly connected thereto,
the blood circuit (10) comprises a first branch circuit (34) and a second branch circuit (35) branching off on an the upstream side of the blood circuit (10) and merging with each other on a downstream side of the blood circuit (10), the first branch circuit (34) being connected to the primary side (60) of the first blood purifier (20) and the second branch circuit (35) being connected to the primary side (60) of the second blood purifier (21), and
supplemental fluid is supplied to the secondary side (61) of the purification membrane (A) of the second blood purifier (21) while the blood is supplied to the primary side (60) of the first blood purifier (20) and supplemental fluid of the primary side (60) of the filtration membrane (A) which has passed through the filtration membrane (A) of the second blood purifier (21) is caused to flow into the upstream and/or downstream of the first branch circuit (34) via the second branch circuit (35).

6. The blood purification device (1) according to any of claims 1 to 5, configured to perform a continuous slow type blood purification process.

7. The blood purification device (1) according to claim 1, comprising:
the plurality of blood purifiers (20, 21), wherein the blood circuit (10) comprises an upstream circuit (32) configured to supply blood collected from a living body to a branch part (31) and a downstream circuit (37) configured to return the blood at a merge part (33) to the living body;
a plurality of branch circuits (34, 35) branching off from the branch part (31) of the upstream circuit (32) toward the respective blood purifiers (20, 21) and merging with each other at the merge part (33) of the downstream circuit (37) via the respective blood purifiers (20, 21);
cleaning fluid supply means (12) configured to selectively supply cleaning fluid to the blood purifiers (20, 21) of the plurality of branch circuits (34, 35); and
a control unit (13) configured to operate, while the blood is supplied from the upstream circuit (32) to the blood purifier (20, 21) of one of the branch circuits (34, 35) and the blood having passed through the blood purifier (20, 21) is caused to flow into the downstream circuit (37), the cleaning fluid supply means (12) to supply the cleaning fluid to the blood purifier (20, 21) of the other one of the branch circuits (34, 35) and to feed the cleaning fluid to the branch part (31) and the merge part (33) via the branch circuit (34, 35) to be supplied to the blood.

8. The blood purification device (1) according to claim 7, configured to supply the cleaning fluid to secondary sides (61) of filtration membranes (A) of the blood purifiers (20, 21), caused to flow out to primary sides (60) via the filtration membranes (A), and fed to the branch part (31) and the merge part (33).

9. The blood purification device (1) according to claim 7 or 8, configured to repeatedly supply the blood to the plurality of blood purifiers (20, 21) in a predetermined order, and to supply the cleaning fluid to the blood purifier (20, 21) to which the blood has been supplied in a previous order.

10. The blood purification device (1) according to any of claims 7 to 9, wherein the control unit (13) is configured to temporarily increase the supply flow rate of the cleaning fluid in the step of supplying the cleaning fluid to the blood purifier (20, 21) of the other one of the branch circuits (34, 35).

11. The blood purification device (1) according to any of claims 7 to 10, wherein the blood purification device (1) is a continuous slow type blood purification device.

## Patentansprüche

1. Blutreinigungsvorrichtung (1), die einen Blutkreislauf (10) aufweist, der so konfiguriert ist, dass er aus einem Körper entnommenes Blut Blutreinigern (20, 21) zuführt und das von den Blutreinigern (20, 21) gereinigte Blut in den Körper zurückführt, umfassend:
eine Vielzahl von Blutreinigern (20, 21), die parallel zum Blutkreislauf (19) geschaltet sind;
eine Schaltereinrichtung (11), die so konfiguriert ist, dass sie das Blut selektiv der Vielzahl von Blutreinigern (20, 21) zuführt; und
eine Reinigungsflüssigkeit-Zuführungseinrichtung (12), die so konfiguriert ist, dass sie Reinigungsflüssigkeit selektiv der Vielzahl von Blutreinigern (20, 21) zuführt;
**dadurch gekennzeichnet, dass**
jeder Blutreiniger (20, 21) eine Filtrationsmembran A mit einer primären Seite (60) und einer sekundären Seite (61) umfasst, wobei die Blutreiniger (20, 21) so mit dem Blutkreislauf (10) verbunden sind, dass das Blut den primären Seiten (60) der Filtrationsmembranen (A) der Blutreiniger (20, 21) zugeführt wird; und
die Reinigungsflüssigkeit-Zuführungseinrichtung (12) so konfiguriert ist, dass sie die Reinigungsflüssigkeit zu den sekundären Seiten (61) der Filtrationsmembranen (A) der Blutreiniger (20, 21) zuführt, wobei die Reinigungsflüssigkeit eine Ergänzungsflüssigkeit ist, die während der Blutreinigung dem Blut zugeführt werden soll.

2. Blutreinigungsvorrichtung (1) gemäß Anspruch 1, wobei der Blutkreislauf (10) eine Vielzahl von Zweigkreisläufen (34, 35) umfasst, die auf einer Anströmseite des Blutkreislaufs (10) abzweigen und an die jeweiligen Blutreiniger (20, 21) anzuschließen sind und auf einer Abströmseite des Blutkreislaufs (10) über die jeweiligen Blutreiniger (20, 21) zusammenlaufen; und
die Schaltereinrichtung (11) eine Pumpe (80, 81) umfasst, die so konfiguriert ist, dass sie Flüssigkeit vorwärts und rückwärts in die jeweiligen Zweigkreisläufe (34, 35) einspeist.

3. Blutreinigungsvorrichtung (1) gemäß Anspruch 1 oder 2, wobei es sich bei der Vielzahl von Blutreinigern (20, 21) um einen ersten und einen zweiten Blutreiniger (20, 21) handelt und wobei der Blutkreislauf (10) den ersten und den zweiten Blutreiniger (20, 21) aufweist, die parallel dazu geschaltet sind,
wobei die Blutreinigungsvorrichtung (1) weiterhin eine Steuerungseinheit (13) umfasst, die so konfiguriert ist, dass sie die Zufuhr des Blutes und die Zufuhr der Reinigungsflüssigkeit so steuert, dass die Reinigungsflüssigkeit dem zweiten Blutreiniger (21) zugeführt wird, während das Blut dem ersten Blutreiniger (20) zugeführt wird, und die Reinigungsflüssigkeit dem ersten Blutreiniger (20) zugeführt wird, während das Blut dem zweiten Blutreiniger (21) zugeführt wird.

4. Blutreinigungsvorrichtung (1) gemäß einem der Ansprüche 1 bis 3, wobei die Reinigungsflüssigkeit-Zuführungseinrichtung (12) so konfiguriert ist, dass sie die Zuführungsfließgeschwindigkeit der Reinigungsflüssigkeit zu den Blutreinigern (20, 21) ändern kann.

5. Blutreinigungsvorrichtung (1) gemäß einem der Ansprüche 1 bis 4, wobei es sich bei der Vielzahl von Blutreinigern (20, 21) um einen ersten und einen zweiten Blutreiniger (20, 21) handelt und wobei der Blutkreislauf (10) den ersten und den zweiten Blutreiniger (20, 21) aufweist, die parallel dazu geschaltet sind,
wobei der Blutkreislauf (10) einen ersten Zweigkreislauf (34) und einen zweiten Zweigkreislauf (35) umfasst, die auf einer Anströmseite des Blutkreislaufs (10) abzweigen und auf einer Abströmseite des Blutkreislaufs (10) zusammenlaufen, wobei der erste Zweigkreislauf (34) mit der primären Seite (60) des ersten Blutreinigers verbunden ist und der zweite Zweigkreislauf (35) mit der primären Seite (60) des zweiten Blutreinigers (21) verbunden ist, und
der sekundären Seite (61) der Reinigungsmembran (A) des zweiten Blutreinigers (21) Ergänzungsflüssigkeit zugeführt wird, während das Blut der primären Seite (60) des ersten Blutreinigers (20) zugeführt wird, und bewirkt wird, dass Ergänzungsflüssigkeit der primären Seite (60) der Filtrationsmembran (A), die durch die Filtrationsmembran (A) des zweiten Blutreinigers (21) geflossen ist, über den zweiten Zweigkreislauf (35) auf die Abström- und/oder Anströmseite des ersten Zweigkreislaufs (34) strömt.

6. Blutreinigungsvorrichtung (1) gemäß einem der Ansprüche 1 bis 5, die so konfiguriert ist, dass sie ein kontinuierliches Blutreinigungsverfahren des langsamen Typs durchführt.

7. Blutreinigungsvorrichtung (1) gemäß Anspruch 1, umfassend:
die Vielzahl von Blutreinigern (20, 21), wobei der Blutkreislauf (10) einen vorgeschalteten Kreislauf (32), der so konfiguriert ist, dass er aus einem lebenden Körper entnommenes Blut einem Abzweigteil (31) zuführt, und einen nachgeschalteten Kreislauf (37), der so konfiguriert ist, dass er das Blut an einem Zusammenlaufteil (33) in den lebenden Körper zurückführt, umfasst;
eine Vielzahl von Zweigkreisläufen (34, 35), die vom Abzweigteil (31) des vorgeschalteten Kreislaufs (32) zu den jeweiligen Blutreinigern (20, 21) hin abzweigen und am Zusammenlaufteil (33) des nachgeschalteten Kreislaufs (37) über die jeweiligen Blutreiniger (20, 21) zusammenlaufen;
eine Reinigungsflüssigkeit-Zuführungseinrichtung (12), die so konfiguriert ist, dass sie Reinigungsflüssigkeit selektiv den Blutreinigern (20, 21) der der Vielzahl von Zweigkreisläufen (34, 35) zuführt; und
eine Steuerungseinheit (13), die so konfiguriert ist, dass sie, während das Blut aus dem vorgeschalteten Kreislauf (32) zu dem Blutreiniger (20, 21) eines der Zweigkreisläufe (34, 35) zugeführt wird und bewirkt wird, dass das Blut, das durch den Blutreiniger (20, 21) geflossen ist, in den nachgeschalteten Kreislauf (37) strömt, die Reinigungsflüssigkeit-Zuführungseinrichtung (12) so ansteuert, dass sie die Reinigungsflüssigkeit dem Blutreiniger (20, 21) des anderen Zweigkreislaufs (34, 35) zuführt und die Reinigungsflüssigkeit über den Zweigkreislauf (34, 35) dem Abzweigteil (31) und dem Zusammenlaufteil (33) zuführt, um sie dem Blut zuzuführen.

8. Blutreinigungsvorrichtung (1) gemäß Anspruch 7, die so konfiguriert ist, dass sie die Reinigungsflüssigkeit sekundären Seiten (61) von Filtrationsmembranen (A) der Blutreiniger (20, 21) zuführt, wo bewirkt wird, dass sie über die Filtrationsmembranen (A) zu primären Seiten (60) hinausfließt, und sie dem Abzweigteil (31) und dem Zusammenlaufteil (33) zugeführt wird.

9. Blutreinigungsvorrichtung (1) gemäß Anspruch 7 oder 8, die so konfiguriert ist, dass sie das Blut der Vielzahl von Blutreinigern (20, 21) wiederholt in einer vorbestimmten Reihenfolge zuführt und dass sie die Reinigungsflüssigkeit demjenigen Blutreiniger (20, 21) zuführt, dem in einer vorherigen Reihenfolge das Blut zugeführt wurde.

10. Blutreinigungsvorrichtung (1) gemäß einem der Ansprüche 7 bis 9, wobei die Steuerungseinheit (13) so konfiguriert ist, dass sie die Zufuhrströmungsgeschwindigkeit der Reinigungsflüssigkeit in dem Schritt des Zuführens der Reinigungsflüssigkeit zum Blutreiniger (20, 21) des anderen Zweigkreislaufs (34, 35) zeitweilig erhöht.

11. Blutreinigungsvorrichtung (1) gemäß einem der Ansprüche 7 bis 10, wobei die Blutreinigungsvorrichtung (1) eine kontinuierliche Blutreinigungsvorrichtung des langsamen Typs ist.

## Revendications

1. Dispositif de purification du sang (1) comportant un circuit de sang (10) qui est configuré de manière à fournir du sang prélevé d'un organisme à des purificateurs de sang (20, 21) et à renvoyer le sang purifié par les purificateurs de sang (20, 21) à l'organisme, comprenant :
une pluralité de purificateurs de sang (20, 21) raccordés en parallèle au circuit de sang (10) ;
des moyens de commutation (11) configurés de manière à fournir le sang, de façon sélective, à la pluralité de purificateurs de sang (20, 21) ; et
des moyens d'alimentation en fluide de nettoyage (12) configurés de manière à fournir du fluide de nettoyage, de façon sélective, à la pluralité de purificateurs de sang (20, 21),
**caractérisé en ce que**
chaque purificateur de sang (20, 21) comprend une membrane de filtration A avec un coté principal (60) et un côté secondaire (61), les purificateurs de sang (20, 21) étant raccordés au circuit de sang (10) de façon à ce que le sang soit fourni aux côtés principaux (60) des membranes de filtration (A) des purificateurs de sang (20, 21), et
les moyens d'alimentation en fluide de nettoyage (12) sont configurés de manière à fournir le fluide de nettoyage aux côtés secondaires (61) des membranes de filtration (A) des purificateurs de sang (20, 21), le fluide de nettoyage étant un fluide supplémentaire à fournir au sang pendant la purification du sang.

2. Dispositif de purification de sang (1) selon la revendication 1, dans lequel le circuit de sang (10) comprend une pluralité de circuits de dérivation (34, 35) se séparant d'un côté amont du circuit de sang (10) pour être raccordés aux purificateurs de sang (20, 21) respectifs et se rejoignant d'un côté aval du circuit de sang (10) via les purificateurs de sang (20, 21) respectifs, et
les moyens de commutation (11) comprennent une pompe (80, 81) configurée de manière à introduire le fluide dans des directions avant et arrière dans les circuits de dérivation (34, 35) respectifs.

3. Dispositif de purification de sang (1) selon la revendication 1 ou 2, dans lequel la pluralité de purificateurs de sang (20, 21) est un premier et un second purificateurs de sang (20, 21) et dans lequel le circuit de sang (10) comporte le premier et le second purificateurs de sang (20, 21) raccordés en parallèle à celui-ci,
le dispositif de purification de sang (1) comprenant en outre une unité de commande (13) configurée pour commander la fourniture du sang et la fourniture du fluide de nettoyage de façon à ce que le fluide de nettoyage soit fourni au second purificateur de sang (21) pendant que le sang est fourni au premier purificateur de sang (20) et que le fluide de nettoyage soit fourni au premier purificateur de sang (20) pendant que le sang est fourni au second purificateur de sang (21).

4. Dispositif de purification de sang (1) selon l'une quelconque des revendications 1 à 3, dans lequel les moyens d'alimentation en fluide de nettoyage (12) sont configurés de manière à modifier le débit d'alimentation du fluide de nettoyage aux purificateurs de sang (20, 21).

5. Dispositif de purification de sang (1) selon l'une quelconque des revendications 1 à 4, dans lequel la pluralité de purificateurs de sang (20, 21) est un premier et un second purificateurs de sang (20, 21) et dans lequel
le circuit de sang (10) comporte le premier et le second purificateur de sang (20, 21) raccordés en parallèle à celui-ci,
le circuit de sang (10) comprend un premier circuit de dérivation (34) et un second circuit de dérivation (35) se séparant d'un côté amont du circuit de sang (10) et se rejoignant d'un côté aval du circuit de sang (10), le premier circuit de dérivation (34) étant raccordé au côté principal (60) du premier purificateur de sang (20) et le second circuit de dérivation (35) étant raccordé au côté principal (60) du second purificateur de sang (21), et
du fluide supplémentaire est fourni au côté secondaire (61) de la membrane de purification (A) du second purificateur de sang (21) pendant que le sang est fourni au côté principal (60) du premier purificateur de sang (20) et du fluide supplémentaire du côté principal (60) de la membrane de filtration (A), qui a traversé la membrane de filtration (A) du second purificateur de sang (21), est amené à s'écouler en amont et/ou en aval du premier circuit de dérivation (34) via le second circuit de dérivation (35).

6. Dispositif de purification de sang (1) selon l'une quelconque des revendications 1 à 5, configuré de manière à effectuer un procédé de purification de sang continu de type lent.

7. Dispositif de purification de sang (1) selon la revendication 1, comprenant :
la pluralité de purificateurs de sang (20, 21), le circuit de sang (10) comprenant un circuit amont (32) configuré de manière à fournir du sang prélevé d'un organisme vivant à une partie de ramification (31) et un circuit aval (37) configuré de manière à renvoyer le sang à l'organisme vivant au niveau d'une partie de jonction (33) ;
une pluralité de circuits de dérivation (34, 35) se séparant à partir de la partie de ramification (31) du circuit amont (32) en direction des purificateurs de sang (20, 21) respectifs et se rejoignant au niveau de la partie de jonction (33) du circuit aval (37) via les purificateurs de sang (20, 21) respectifs ;
des moyens d'alimentation en fluide de nettoyage (12) configurés de manière à fournir du fluide de nettoyage, de façon sélective, aux purificateurs de sang (20, 21) de la pluralité de circuits de dérivation (34, 35) ; et
une unité de commande (13) configurée de manière à, pendant que le sang est fourni au purificateur de sang (20, 21) de l'un des circuits de dérivation (34, 35) depuis le circuit amont (32), et que le sang ayant traversé le purificateur de sang (20, 21) est amené à s'écouler dans le circuit aval (37), actionner les moyens d'alimentation en fluide de nettoyage (12) pour fournir le fluide de nettoyage au purificateur de sang (20, 21) de l'autre des circuits de dérivation (34, 35) et pour introduire le fluide de nettoyage à fournir au sang dans la partie de ramification (31) et a partie de jonction (33) via le circuit de dérivation (34, 35).

8. Dispositif de purification de sang (1) selon la revendication 7, configuré de manière à fournir le fluide de nettoyage aux côtés secondaires (61) des membranes de filtration (A) des purificateurs de sang (20, 21), amené à s'écouler hors des côtés principaux (60) via les membranes de filtration (A), et introduit dans la partie de ramification (31) et à la partie de jonction (33).

9. Dispositif de purification de sang (1) selon la revendication 7 ou 8, configuré de manière à fournir de manière répétitive le sang à la pluralité de purificateurs de sang (20, 21) dans un ordre prédéterminé, et à fournir le fluide de nettoyage au purificateur de sang (20, 21) auquel le sang a été fourni dans un ordre précédent.

10. Dispositif de purification de sang (1) selon l'une quelconque des revendications 7 à 9, dans lequel le dispositif de commande (13) est configuré de manière à augmenter temporairement le débit d'alimentation du fluide de nettoyage lors de l'étape de fourniture du fluide de nettoyage au purificateur de sang (20, 21) de l'autre des circuits de dérivation (34, 35).

11. Dispositif de purification de sang (1) selon l'une quelconque des revendications 7 à 10, le dispositif de purification de sang (1) étant un dispositif de purification de sang continu de type lent.
